# EUROPÄISCHE PATENTSCHRIFT

(11) **EP 1 983 456 B1**
(45) Veröffentlichungstag und Bekanntmachung des Hinweises auf die Patenterteilung: **14.08.2013**
(21) Anmeldenummer: 07007991.8
(22) Anmeldetag: 19.04.2007
(51) Int. Cl.: G06F 19/00

(54) **Verfahren zum Einstellen eines Basalratenprofils für eine Insulinpumpe**
Method for adjusting a base rate profile for an insulin pump
Procédé de réglage d'un profile de basalt pour une pompe à insuline

(43) Veröffentlichungstag der Anmeldung: 22.10.2008
(73) Patentinhaber: F.Hoffmann-La Roche AG, 4070 Basel (CH); Roche Diagnostics GmbH, 68305 Mannheim (DE)
(72) Erfinder: Vering, Thomas, 3047 Bremgarten b. Bern (CH); Haueter, Ulrich, 3506 Grosshöchstetten (CH); von Büren, Daniel, 68300 Saint-Louis (FR)
(74) Vertreter: Rentsch Partner AG

(56) Entgegenhaltungen:
- EP-A- 0 002 776
- EP-A- 0 164 904
- WO-A-96/36389
- US-A- 5 088 981
- US-A1- 2004 055 611
- US-A1- 2005 272 640
- US-A1- 2006 009 734

## Beschreibung

Die Erfindung betrifft ein Verfahren zum Einstellen eines Basalratenprofils für eine Insulinpumpe gemäss Oberbegriff des Anspruchs 1.

Aus der Patentschrift US 6,810,290 B2 ist ein Verfahren zum Einstellen von Basalraten für eine implantierbare Insulinpumpe bekannt, bei dem bei einer Veränderung der von der Insulinpumpe abzugebenden Basalraten nicht sämtliche Basalraten, sondern nur die zu verändernden Basalraten neu eingestellt werden müssen. Die Neueinstellung erfolgt durch Spezifizierung der Basalrate und des Startabgabezeitpunkts.

Es ist Aufgabe der Erfindung, ein einfaches Verfahren zum Einstellen eines physiologischen Basalratenprofils für eine Insulinpumpe bereitzustellen. Unter einem physiologischen Basalratenprofil wird insbesondere ein möglichst stetiges Abgabeprofil verstanden.

Die Aufgabe wird durch ein Verfahren mit den Merkmalen des Anspruchs 1 gelöst. Das erfindungsgemässe Verfahren zum Einstellen eines Basalratenprofils für eine Insulinpumpe mit einer Eingabeeinheit und einer Berechnungseinheit kennzeichnet sich dadurch aus, dass eine Anzahl von Stützstellen für das Basalratenprofil über die Eingabeeinheit der Insulinpumpe definiert, eine kontinuierliche Funktion zu den Stützstellen, die die Stützstellen und vorzugsweise gegebenenfalls bereits hinterlegte Basalraten eines hinterlegten Basalratenprofils abbildet, mittels der Berechnungseinheit der Insulinpumpe gebildet und eine zeitliche Folge von von der Insulinpumpe während bestimmter Zeitabschnitte abzugebender Basalraten aus der kontinuierlichen Funktion mittels der Berechnungseinheit der Insulinpumpe erzeugt wird. Die zeitliche Folge stellt das Basalratenprofil dar. Das Basalratenprofil ist vorzugsweise in einer Speichereinheit hinterlegt, die der Insulinpumpe zugeordnet ist und bevorzugt einen Teil der Insulinpumpe bildet. Unter dem Ausdruck "Einstellen eines Basalratenprofils" kann auch das Erzeugen bzw. Hinterlegen eines Basalratenprofils verstanden.

Eine Stützstelle ist durch den Startzeitpunkt einer Basalratenabgabe und der diesem Startzeitpunkt zugeordneten abzugebenden Basalrate oder der diesem Startzeitpunkt zugeordneten absoluten bzw. relativen abzugebenden Insulinmenge definiert. Als Basalrate gilt die Insulinmenge, die benötigt wird, um den Zuckerstoffwechsel eines Patienten - ohne zusätzliche Beeinflussung durch Nahrungsaufnahme bzw. aussergewöhnliche, Glucose verbrauchende Aktivitäten wie beispielsweise sportliche Aktivitäten - stabil zu halten. Die Eingabeeinheit und die Berechnungseinheit der Insulinpumpe können sowohl ausserhalb der Insulinpumpe als auch in der Insulinpumpe selbst angeordnet sein.

Mittels des erfindungsgemässen Verfahrens ist es möglich, ein vollständiges physiologisches Basalratenprofil einzustellen, wie dies beispielsweise zu Therapiebeginn oder bei grösserer Umstellung der Therapie und somit im Wesentlichen kompletter Änderung des Basalratenprofils der Fall ist. Ferner können mittels des erfindungsgemässen Verfahrens bei einem bereits hinterlegten Basalratenprofil durch Definieren einzelner Stützstellen oder einer einzigen Stützstelle auf einfache Weise physiologisch korrekte Änderungen bzw. Anpassungen des hinterlegten Basalratenprofils erzielt werden. Beispielsweise kann auf diese Weise ein Finetuning des hinterlegten Basalratenprofils durchgeführt werden.

Ein Basalratenprofil deckt üblicherweise einen Zeitbereich von 24 Stunden ab, wobei typischerweise jeder Stunde eine Basalrate zugeordnet ist, sodass das Basalratenprofil aus einer zeitlichen Folge von 24 Basalraten gebildet wird. Alternativ kann auch jeder halben Stunde eine Basalrate zugeordnet sein, sodass das Basalratenprofil aus einer zeitlichen Folge von 48 Basalraten gebildet wird. Entsprechend kann eine solche zeitliche Folge auch aus einer anderen Anzahl von Basalraten, wie beispielsweise 12 oder 96 Basalraten, gebildet sein.

Gemäss Ausgestaltung der Erfindung werden vier bis zehn Stützstellen für das Basalratenprofil definiert. Das Definieren der Stützstellen erfolgt durch Eingeben mittels der Eingabeeinheit der Insulinpumpe, wobei vorzugsweise für jede zu definierende Stützstelle der Startzeitpunkt einer Basalratenabgabe und die diesem Startzeitpunkt zugeordneten abzugebenden Basalrate eingegeben werden. Die Stützstellen werden in der Regel für ein Basalratenprofil definiert, welches einen Zeitbereich von 24 Stunden abdeckt. Es können jedoch auch Stützstellen für ein Basalratenprofil definiert werden, welches einen Zeitbereich von mehreren Tagen, beispielsweise eine Woche oder einen Monat wie dies z.B. bei einem biorhythmischen Profil der Fall sein kann, abdeckt. Die Anzahl der definierten Stützstellen kann dann entsprechend erhöht werden.

Zu den Stützstellen wird vorzugsweise mittels Interpolation und/oder Approximation eine kontinuierliche Funktion gebildet, die die Stützstellen bzw. die Stützstellen und gegebenenfalls bereits hinterlegte Basalraten abbildet. Das heisst, die Stützstellen bzw. die Stützstellen und gegebenenfalls bereits hinterlegte Basalraten werden durch eine geeignete kontinuierliche Kurve miteinander verbunden. Es kann beispielsweise die lineare Interpolation eingesetzt werden, bei der als Ansatzfunktion ein lineares Polynom zur Lösung des zugrunde liegenden Interpolationsproblems gewählt wird. Vorzugsweise wird die trigonometrische Interpolation eingesetzt, bei der als Ansatzfunktion ein trigonometrisches Polynom, beispielsweise eine Sinus- und/oder Cosinus-Funktion, als Funktion zur Verbindung der Stützstellen verwendet wird. Durch die Anwendung von trigonometrischer Interpolation kann man eine stetige bzw. harmonische kontinuierliche Funktion und somit eine im Wesentlichen stetigere bzw. harmonischere Änderung der Basalrate erhalten. Als Ansatzfunktionen für die Interpolation können ferner auch so genannte Splines eingesetzt werden.

Zum Erzeugen einer zeitlichen Funktion von Basalraten aus der kontinuierlichen Funktion wird die kontinuierliche Funktion mittels der Berechnungseinheit diskretisiert. Bei der Diskretisierung wird der Zeitbereich der kontinuierlichen Funktion in eine endliche Zahl aneinander angrenzender Zeitabschnitte aufgeteilt, wobei jedem Zeitabschnitt ein Wert der kontinuierlichen Funktion zugeordnet wird. Bei dem zugeordneten Werte der kontinuierlichen Funktion kann es sich beispielsweise um den Wert zu Beginn des jeweiligen Zeitabschnitts oder um den in der Mitte des jeweiligen Zeitabschnitts liegenden Wert handeln. Der dem jeweiligen Zeitabschnitt zugeordnete Wert bildet die während des jeweiligen Zeitabschnitts von der Insulinpumpe an den Patienten abzugebende Basalrate. Die Anzahl der Basalraten der erzeugten zeitlichen Folge ist vorzugsweise grösser als die Anzahl der definierten bzw. der eingegebenen Stützstellen. Das erfindungsgemässe Verfahren erzeugt dann vorteilhafterweise aus einigen eingegebenen Stützstellen - und gegebenenfalls zusätzlich aus bereits hinterlegten Basalraten - ein Basalratenprofil, das auch Basalraten enthält, die zwischen den durch die Stützstellen gebildeten Basalraten liegen. Das heisst, mittels des erfindungsgemässen Verfahrens werden zwischen den Stützstellen liegende Zwischenwerte für Basalraten generiert.

Während der jeweiligen Zeitabschnitte bleibt die abzugebende Basalrate konstant. Die abzugebende Basalrate ist bevorzugt in der Einheit IE/Stunden definiert, wobei 1 IE 41,67 µg Insulin (hochrein) bzw. 35 µg Insulin (wasserfrei) entspricht. Bei der Einheit IE handelt es sich um die internationale Einheit für Stoffmengen, die im englischsprachigen Raum mit IU (international unit) bezeichnet wird.

Durch das Erzeugen einer zeitlichen Folge mittels Diskretisierung aus der kontinuierlichen Funktion ist es möglich, dem Patienten bzw. Benutzer der Insulinpumpe die momentan abgegebene Insulinmenge beispielsweise über eine gegebenenfalls der Insulinpumpe zugeordnete Anzeigeeinheit, auch Display genannt, anzuzeigen, da eine derartige Anzeigeeinheit häufig nur diskrete Werte und keine kontinuierlichen Funktionen anzeigen kann.

Die zeitliche Folge von abzugebenden Basalraten kann 24 Basalraten umfassen, wobei jede Basalrate über einen Zeitraum von 1 Stunde von der Insulinpumpe abgegeben werden soll. Es können jedoch auch weniger als 24 Basalraten bei der Erzeugung der zeitlichen Folge generiert werden, sodass beispielsweise eine Basalrate jeweils über einen längeren Zeitraum als 1 Stunde abgegeben wird. Es können auch mehr als 24 Basalraten erzeugt werden, sodass bei einem Gesamtabgabezeitbereich von 24 Stunden eine Basalrate beispielsweise für weniger als 1 Stunde abgegeben wird. Die Zeitabschnitte, in denen die Basalraten an den Patienten abzugeben sind, können äquidistant sein, sie können jedoch auch unterschiedlich lang sein, sodass beispielsweise eine erste Basalrate der zeitlichen Folge für 15 Minuten abgegeben wird, während eine zweite Basalrate für 3 Stunden abgegeben wird.

Die kontinuierliche Funktion zu den Stützstellen wird vorzugsweise derart gebildet, dass eine Stützstelle, die wertemässig zwischen zwei tiefer liegenden Stützstellen liegt, ein Maximum der kontinuierlichen Folge bildet, während eine Stützstelle, die zwischen zwei wertemässig höher liegenden Stützstellen liegt, ein Minimum der kontinuierlichen Funktion bildet. Auf diese Weise kann vorteilhafterweise ein Über- bzw. Unterschwingen bei der Interpolation, wie dies beispielsweise bei einer Interpolation mittels einer Spline-Funktion auftreten könnte, verhindert werden. Ferner werden bei der Bildung der kontinuierlichen Funktion Stützstellen mit gleichen Werten vorzugsweise mittels einer Geraden, d.h. horizontal, verbunden. Weisen benachbarte Stützstellen unterschiedliche Werte auf und definiert eine Stützstelle ein Minimum und die benachbarte Stützstelle ein Maximum der kontinuierlichen Funktion, so weist die kontinuierliche Funktion zwischen dem Minimum und dem Maximum vorzugsweise genau einen Wendepunkt auf. Der Wendepunkt liegt bevorzugt sowohl zeitlich als auch amplitudenmässig, wobei die Amplitude insbesondere der abzugebenden Basalrate entspricht, in der Mitte zwischen den beiden Stützstellen.

Die Zeitabschnitte, in die die kontinuierliche Funktion zur Bildung der zeitlichen Folge unterteilt wird, sind bevorzugt derart gewählt, dass bei den Zeitabschnitten, bei denen die kontinuierliche Funktion durch eine Stützstelle verläuft, die Stützstelle zeitmässig in der Mitte des Zeitabschnitts positioniert ist.

Beim Bilden der kontinuierlichen Funktion werden die bezüglich ihres Zeitpunkts letzte Stützstelle oder letzte bereits hinterlegte Basalrate und die bezüglich ihres Zeitpunkts erste Stützstelle oder erste bereits hinterlegte Basalrate durch die kontinuierliche Funktion direkt verbunden, sodass keine unstetige bzw. unphysiologische Basalratenabgabe erfolgt bzw. die kontinuierliche Funktion keinen Sprung aufweist. Die zeitlich letzte und die zeitliche erste Stützstelle bzw. hinterlegte Basalrate werden auch als Wrap-around-Punkte bezeichnet. Der der ersten Basalrate der zeitlichen Folge zugewiesene Zeitabschnitt folgt somit zeitlich unmittelbar auf den der letzten Basalrate zugewiesenen Zeitabschnitt. Das von der zeitlichen Folge von Basalraten gebildete Basalratenprofil wird somit ununterbrochen und immer wieder aufeinanderfolgend durchlaufen. Der Zeitbereich des Basalratenprofils (bzw. dessen Zeitskala) kann dann, beispielsweise anstatt von 0 Uhr bis 0 Uhr auch von 5 Uhr bis 5 Uhr verlaufend oder gemäss einem beliebigen anderen Zeitbereich gewählt und auf einem vorzugsweise vorgesehenen Display dem Benutzer angezeigt werden. Bei einer Zeitskala von 5 Uhr morgens bis 5 Uhr morgens kann die gesamte Nacht und der ganze Tag jeweils als zusammenhängendes Basalratenprofil dargestellt werden.

Ändert sich die Infusionsmenge, die einem Patienten während beispielsweise eines Tages, einer Woche oder eines Monats verabreicht werden soll, so können die einzelnen Basalraten des Basalratenprofils entsprechend der Infusionsmengenänderung proportional angepasst werden. Dies kann beispielsweise in der Art erfolgen, dass die Stützstellen proportional angepasst werden, aus diesen proportional angepassten Stützstellen eine kontinuierliche Funktion gebildet wird und aus dieser kontinuierlichen Funktion wiederum durch Diskretisierung eine zeitliche Folge von Basalraten, d.h. ein neues Basalratenprofil, erzeugt wird. Auf diese Weise ist ein physiologisch adäquates und gleichzeitiges Anpassen sämtlicher Basalraten oder auch sämtlicher hinterlegter Basalratenprofile möglich. Die proportionale Anpassung der Stützstellen kann durch Multiplikation der Werte der Stützstellen mit einem Faktor erreicht werden, der dem Verhältnis zwischen der bisherigen Gesamtinfusionsmenge und der neuen Gesamtinfusionsmenge über den Zeitbereich des Basalratenprofils entspricht.

Ein oder mehrere mittels des erfindungsgemässen Verfahrens eingestellte bzw. hinterlegte Basalratenprofile können bezüglich ihrer jeweiligen Amplitude bzw. Profiltiefe angepasst werden, indem durch erneute Anwendung des erfindungsgemässen Verfahrens eine oder mehrere Stützstellen neu eingegeben werden, aus diesen neu eingegebenen Stützstellen und den bereits hinterlegten unveränderten Stützstellen und/oder den bereits hinterlegten Basalraten der hinterlegten zeitlichen Folge eine neue kontinuierliche Funktion gebildet und aus dieser wiederum eine neue zeitliche Folge von Basalraten erzeugt wird. Hierbei kann die Amplitude des Basalratenprofils durch entsprechende Änderung der Werte der Stützstellen vorteilhafterweise in der Art geändert werden, dass die gesamte Infusionsmenge während des Zeitbereichs des Basalratenprofils konstant bleibt. Werden bereits hinterlegte Basalraten zusammen mit neu eingegebenen Stützstellen für die Bildung einer neuen zeitlichen Folge von Basalraten verwendet, so werden vorzugsweise diejenigen hinterlegten Basalraten nicht verwendet, die denjenigen Zeitpunkten zugeordnet sind, denen die neu eingegebenen Stützstellen zugeordnet sind.

Weiter kann ein bereits mit Hilfe des erfindungsgemässen Verfahrens eingestelltes bzw. eingegebenes Basalratenprofil auf der Zeitskala bzw. Zeitachse vor-und zurückverschoben werden, ohne dass sich die Gesamtinfusionsmenge ändert. Auf diese Weise können physiologisch bedingte Insulin-Wirkverzögerungen kompensiert werden. Zur Bewirkung eines derartigen Verschiebens weist die Eingabeeinheit der Insulinpumpe vorzugsweise entsprechende Tasten auf, sodass vorzugsweise durch Betätigung einer Taste gemäss einem unverschobenen Basalratenprofil Insulin verabreicht wird, während bei Betätigung einer anderen Taste Insulin gemäss einem um einen bestimmten Zeitraum, beispielsweise eine Stunde, vorverschobenen Basalratenprofil verabreicht wird. Das unverschobene Basalratenprofil entspricht dabei einem Normalmodus.

Wird das erfindungsgemässe Verfahren eingesetzt, um ein bereits hinterlegtes Basalratenprofil neu einzustellen bzw. zu ändern, so kann die Definition der Stützstellen dadurch erfolgen, dass prozentuale Änderungen von zumindest einigen der bereits hinterlegten bzw. definierten Stützstellen und/oder der bereits hinterlegten Basalraten über die Eingabeeinheit der Insulinpumpe eingegeben werden bzw. dass eine neue Gesamtinfusionsmenge, die während des Zeitbereichs bzw. der Zeitdauer des Basalratenprofils abgegeben werden soll, eingegeben wird, wobei über die prozentuale Abweichung der neuen Gesamtinfusionsmenge gegenüber einer bereits hinterlegten Gesamtinfusionsmenge neue bzw. geänderte Stützstellen definiert werden.

Gemäss besonderer Ausgestaltung des erfindungsgemässen Verfahrens wird die physiologische Plausibilität des eingestellten Basalratenprofils dadurch ermittelt, dass die Anzahl der Vorzeichenwechsel der Ableitung der kontinuierlichen Funktion durch die Berechnungseinheit der Infusionspumpe bestimmt wird und für den Fall, dass die Anzahl der Vorzeichenwechsel einen vordefinierten Grenzwert überschreitet, ein Warnsignal erzeugt wird und/oder die Abgabe von Insulin gemäss dem Basalratenprofil unterbunden wird. Der vordefinierte Grenzwert kann von einem Benutzer beispielsweise vor der ersten Einstellung eines Basalratenprofils mittels der Eingabeeinheit der Infusionspumpe hinterlegt bzw. eingegeben worden sein. Beispielhaft kann der vordefinierte Grenzwert für die Anzahl der erlaubten Vorzeichenwechsel 5 betragen.

Gemäss weiterer bevorzugter Ausgestaltung der Erfindung werden die (neu) definierten Stützstellen mit bereits vordefinierten bzw. hinterlegten Stützstellen und/oder mit bereits hinterlegten Basalraten der bereits hinterlegten zeitlichen Folge, die den neu definierten Stützstellen entsprechen, beispielsweise eines bereits hinterlegten Basalratenprofils, verglichen und nur für den Fall, dass die definierten Stützstellen um nicht mehr als einen vordefinierten Wert, insbesondere um nicht mehr als 20 %, von den vordefinierten bzw. hinterlegten Stützstellen und/oder von den hinterlegten Basalraten abweichen, wird eine kontinuierliche Funktion gebildet und eine zeitliche Folge abzugebender Basalraten wird aus der kontinuierlichen Funktion erzeugt. Es kann ein Warnsignal erzeugt werden, wenn die definierten Stützstellen um mehr als den vordefinierten Wert, insbesondere um mehr als 20 %, von den vordefinierten Stützstellen und/oder den hinterlegten Basalraten abweichen. Es kann einerseits vorgesehen sein, dass jede neu definierte Stützstelle um nicht mehr als 20 % von der ihr entsprechenden vordefinierten bzw. hinterlegten Stützstelle oder hinterlegten Basalrate abweichen darf. Alternativ kann vorgesehen sein, dass sämtliche neu definierten Stützstellen, also die Summe ihrer Werte, um nicht mehr als 20 % von den hinterlegten Stützstellen und/oder den hinterlegten Basalraten, die den neu definierten Stützstellen entsprechen, d.h. der Summe von deren Werten, abweichen dürfen.

Dies führt zu erhöhter Sicherheit bei der Verwendung der Infusionspumpe, indem vor physiologisch unüblichen Einstellungen gewarnt wird bzw. ein physiologisch unübliches Basalratenprofil nicht zum Einsatz kommt.

Weitere vorteilhafte Ausgestaltungen der Erfindung ergeben sich aus den Unteransprüchen und den anhand der Zeichnungen nachfolgend dargestellten Ausführungsbeispielen. Es zeigen:
Figur 1 eine Flussdiagrammdarstellung des erfindungsgemässen Verfahrens,
Figur 2 einen Kurvenverlauf eines mit dem erfindungsgemässen Verfahren eingestellten Basalratenprofils,
Figur 3 einen Kurvenverlauf eines weiteren mit dem erfindungsgemässen Verfahren eingestellten Basalratenprofils,
Figur 4 den Kurvenverlauf gemäss Figur 3 mit neu eingegebener bzw. veränderter zweiter Stützstelle.

In den Figuren bezeichnen gleiche Bezugszeichen strukturell bzw. funktionell gleichwirkende Komponenten.

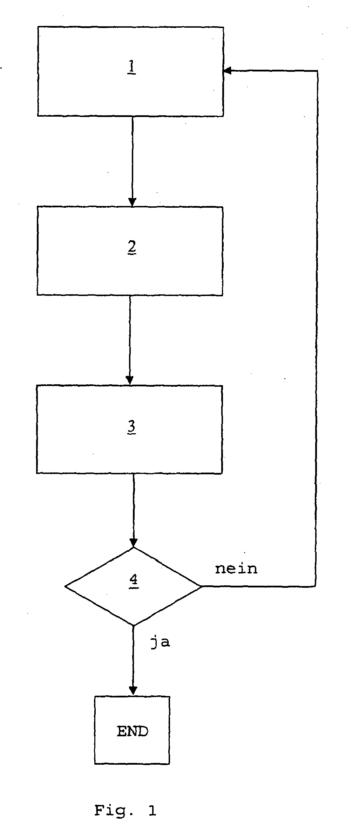

Figur 1 zeigt eine Flussdiagrammdarstellung des erfindungsgemässen Verfahrens. In einem ersten Verfahrensschritt 1 wird eine Anzahl von Stützstellen für das einzustellende Basalratenprofil über eine Eingabeeinheit einer Insulinpumpe definiert. Hierzu werden die abzugebende Basalrate und der Startzeitpunkt für die Abgabe der Basalrate eingegeben bzw. definiert. In einem zweiten Verfahrensschritt 2 wird mittels einer Berechnungseinheit der Insulinpumpe - gegebenenfalls unter Berücksichtigung bereits hinterlegter Basalraten eines bereits hinterlegten Basalratenprofils - eine kontinuierliche Funktion zu den Stützstellen gebildet, die die Stützstellen abbildet. Hierzu werden vorzugsweise Interpolations- bzw. Approximationsverfahren eingesetzt. In einem dritten Verfahrensschritt 3 wird das Basalratenprofil als eine zeitliche Folge von von der Insulinpumpe während bestimmter Zeitabschnitte abzugebender Basalraten aus der kontinuierlichen Funktion mittels der Berechnungseinheit der Insulinpumpe gebildet. Das Basalratenprofil kann dann auf einem vorzugsweise vorgesehenen Display dem Benutzer angezeigt und in einem Verfahrensschritt 4 überprüft werden. Entspricht das Basalratenprofil nicht oder nicht mehr den Vorstellungen des Benutzers bzw. will der Benutzer das Basalratenprofil weiter verändern, so wird zum ersten Verfahrensschritt 1 gesprungen und der Benutzer kann über eine erneute Eingabe von einer oder mehreren Stützstellen das Basalratenprofil verändern, bis ein Basalratenprofil erzeugt worden ist, welches seinen Vorstellungen entspricht (Verfahrensschritt "End"). Die Änderung eines hinterlegten Basalratenprofils kann auch durch Multiplikation des hinterlegten Basalratenprofils mit einem von Benutzer vorgegebenen bzw. einzugebenden Faktor erfolgen, sodass die neuen Stützstellen bzw. den mit diesem Faktor multiplizierten, hinterlegten Stützstellen bzw. Basalraten entsprechen.

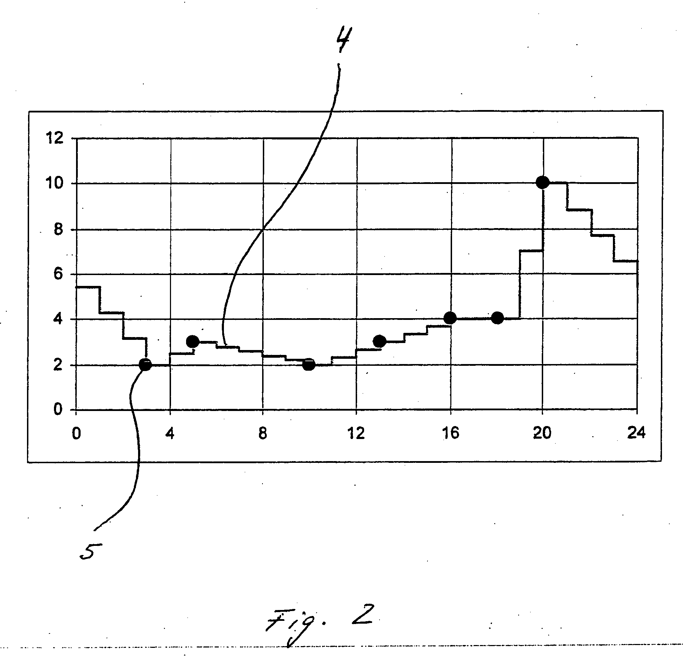

Figur 2 zeigt ein mittels des erfindungsgemässen Verfahrens eingestelltes Basalratenprofil 4, das aus Stützstellen 5 erzeugt worden ist. Zur Bildung der kontinuierlichen Funktion im zweiten Verfahrensschritt 2 wurde lineare Interpolation eingesetzt, d.h. die Stützstellen 5 wurden bei der Interpolation durch lineare Polynome, in diesem Fall durch lineare Polynome erster Ordnung, d.h. Geraden, verbunden. Auf der Abszisse ist die Zeit in Stunden und auf der Ordinate ist die Infusionsmenge in IE (Internationale Einheit, ein IE entspricht 41,67 µg Insulin (hochrein) bzw. 35 µg Insulin (wasserfrei)) aufgetragen. Bei der Diskretisierung der kontinuierlichen Funktion im dritten Verfahrensschritt 3 wurde der Zeitbereich von 24 Stunden in Zeitabschnitte von je 1 Stunde aufgeteilt und jedem dieser Zeitabschnitte wurde der Wert der Basalrate am Anfang des jeweiligen Zeitabschnitts zugewiesen. Auf diese Weise wird die in Figur 2 dargestellte Schrittfunktion als Basalratenprofil erhalten. Durch den Basalratenwert an einer Stützstelle wird vorzugsweise jeweils der Basalratenwert für den gesamten dieser Stützstelle zugeordneten Zeitabschnitt definiert.

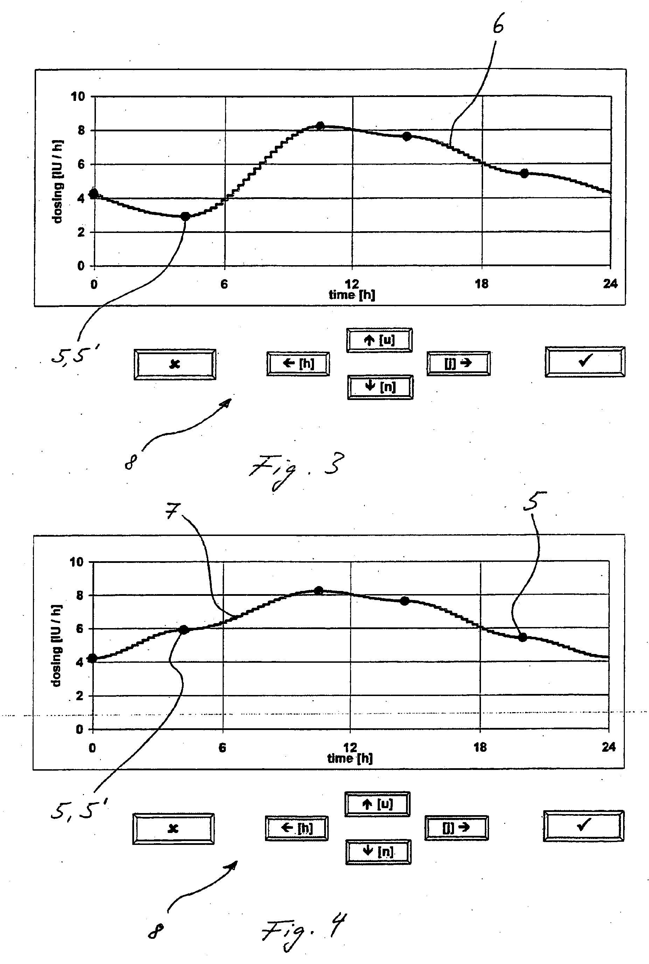

Bei den in den Figuren 3 und 4 dargestellten Kurvenverläufen von Basalratenprofilen wurden trigonometrische Polynome, insbesondere eine Sinus- und/oder Cosinus-Funktion, für die Interpolation zur Bildung einer kontinuierlichen Funktion aus den jeweiligen Stützstellen 5 eingesetzt. Auf der Abszisse ist in den Figuren 3 und 4 wiederum die Zeit in Stunden und auf der Ordinate ist wiederum die Basalrate in der internationalen Einheit IE, die im englischsprachigen Raum der Einheit IU (International Unit) entspricht, dargestellt.

Bei dem in der Figur 4 dargestellten Basalratenprofil 7 wurde gegenüber dem in Figur 3 dargestellten Basalratenprofil 6 die zeitlich gesehen zweite Stützstelle 5' wertemässig gesehen nach oben verschoben bzw. die ursprüngliche zweite Stützstelle 5' gelöscht und eine neue Stützstelle 5' eingegeben. Dies entspricht einer Neudefinition der zweiten Stützstelle 5' gemäss dem ersten Verfahrensschritt 1 des erfindungsgemässen Verfahrens. Im zweiten Verfahrensschritt 2 wird dann die kontinuierliche Funktion zu den Stützstellen 5, 5' oder zu der neuen Stützstelle 5' und den bereits hinterlegten Basalraten, die nicht der ursprünglichen zweiten Stützstelle 5' entsprechen, neu berechnet. In dem dritten Verfahrensschritt 3 wird dann aus der kontinuierlichen Funktion das Basalratenprofil 7 als zeitliche Folge von zu bestimmten Zeitabschnitten abzugebenden Basalraten erzeugt. Bei den Kurvenverläufen in den Figuren 3 und 4 sind die für die Diskretisierung im dritten Verfahrensschritt 3 gewählten Zeitabschnitte kürzer gewählt als beim Kurvenverlauf gemäss Figur 2.

Vorzugsweise weist die Eingabeeinheit der Insulinpumpe Tasten 8 auf, über die eine Stützstelle 5, 5' für das Basalratenprofil 6, 7 gelöscht werden kann (Lösch-Taste "x"), über die eine Stützstelle 5, 5' für das Basalratenprofil 6, 7 eingegeben werden kann (mit einem Haken versehene Taste), über die eine Stützstelle 5, 5' nach oben bzw. nach unten verschoben werden kann (Tasten "u" bzw. "n") und über die das gesamte Basalratenprofil 6, 7 bzw. dessen Stützstellen 5, 5' zeitlich gesehen nach links bzw. nach rechts verschoben werden kann (Tasten "h" und "j"). Wird ein neues Basalratenprofil insbesondere aus neu eingegebenen Stützstellen 5, 5', denen keine bereits hinterlegten Stützstellen 5, 5' entsprechen, und gegebenenfalls bereits hinterlegten Basalraten berechnet, so kann auf die Lösch-Taste verzichtet werden.

## Patentansprüche

1. Verfahren zum Einstellen eines Basalratenprofils (4, 6, 7) für eine Insulinpumpe mit einer Eingabeeinheit und einer Berechnungseinheit, **gekennzeichnet durch** die folgenden Schritte:
- Definieren einer Anzahl von Stützstellen (5, 5') für das Basalratenprofil (4, 6, 7) über die Eingabeeinheit der Insulinpumpe, wobei jede Stützstelle (5, 5') **durch** einen Startzeitpunkt einer Basalratenabgabe und einer diesem Startzeitpunkt zugeordneten abzugebenden Basalrate oder einer diesem Startzeitpunkt zugeordneten abzugebenden Insulinmenge definiert ist,
- Bilden einer kontinuierlichen Funktion zu den Stützstellen (5, 5') mittels Interpolation und/oder Approximation **durch** die Berechnungseinheit der Insulinpumpe, wobei die kontinuierliche Funktion die Stützstellen (5, 5') abbildet und miteinander verbindet, und
- Erzeugen einer zeitlichen Folge (4, 6, 7) von von der Insulinpumpe während bestimmter Zeitabschnitte abzugebenden Basalraten aus der kontinuierlichen Funktion mittels der Berechnungseinheit der Insulinpumpe, indem die kontinuierliche Funktion diskretisiert wird, wobei bei der Diskretisierung der Zeitbereich der kontinuierlichen Funktion in eine endliche Zahl aneinander angrenzender Zeitabschnitte aufgeteilt und jedem Zeitabschnitt ein Wert der kontinuierlichen Funktion zugeordnet wird.

2. Verfahren nach Anspruch 1, **dadurch gekennzeichnet, dass** die kontinuierliche Funktion zusätzlich zu den Stützstellen (5, 5') hinterlegte Basalraten abbildet.

3. Verfahren nach Anspruch 1 oder 2, **dadurch gekennzeichnet, dass** vier bis zehn Stützstellen (5, 5') definiert werden.

4. Verfahren nach einem der vorhergehenden Ansprüche, **dadurch gekennzeichnet, dass** wenigstens 24 Basalraten erzeugt werden.

5. Verfahren nach einem der Ansprüche 1 bis 3, **dadurch gekennzeichnet, dass** weniger als 24 Basalraten erzeugt werden, wobei die Anzahl der erzeugten Basalraten grösser ist als die Anzahl der definierten Stützstellen (5, 5').

6. Verfahren nach einem der vorhergehenden Ansprüche, **dadurch gekennzeichnet, dass** beim Bilden der kontinuierlichen Funktion die bezüglich ihres Zeitpunkts letzte Stützstelle und die bezüglich ihres Zeitpunkts erste Stützstelle durch die kontinuierliche Funktion direkt miteinander verbunden werden, sodass keine unstetige Basalratenabgabe erfolgt.

7. Verfahren nach einem der vorhergehenden Ansprüche, **dadurch gekennzeichnet, dass** die Anzahl der Vorzeichenwechsel der Ableitung der kontinuierlichen Funktion durch die Berechnungseinheit bestimmt wird und für den Fall, dass die Anzahl der Vorzeichenwechsel einen vordefinierten Grenzwert überschreitet, ein Warnsignal erzeugt wird.

8. Verfahren nach einem der vorhergehenden Ansprüche, **dadurch gekennzeichnet, dass** die definierten Stützstellen (5, 5') mit vordefinierten Stützstellen und/oder hinterlegten Basalraten eines hinterlegten Basalratenprofils verglichen werden und nur für den Fall, dass die definierten Stützstellen (5, 5') um nicht mehr als einen vordefinierten Wert, insbesondere um nicht mehr als 20 Prozent, von den vordefinierten Stützstellen und/oder hinterlegten Basalraten abweichen, eine kontinuierliche Funktion zu den definierten Stützstellen (5, 5') und gegebenenfalls hinterlegten Basalraten, die die definierten Stützstellen (5, 5') und gegebenenfalls hinterlegte Basalraten abbildet, mittels der Berechnungseinheit der Insulinpumpe gebildet und eine zeitliche Folge (4, 6, 7) von von der Insulinpumpe während bestimmter Zeitabschnitte abzugebenden Basalraten aus der kontinuierlichen Funktion mittels der Berechnungseinheit der Insulinpumpe erzeugt wird.

9. Verfahren nach einem der vorhergehenden Ansprüche, **dadurch gekennzeichnet, dass** die definierten Stützstellen (5, 5') mit vordefinierten Stützstellen und/oder hinterlegten Basalraten verglichen werden und für den Fall, dass die definierten Stützstellen (5, 5') um mehr als einen vordefinierten Wert, insbesondere um mehr als 20 Prozent, von den vordefinierten Stützstellen und/oder hinterlegten Basalraten abweichen, ein Warnsignal erzeugt wird.

## Claims

1. A method for adjusting a basal rate profile (4, 6, 7) for an insulin pump having an input unit and a calculation unit, **characterized by** the following steps:
- Definition of a number of node points (5, 5') for the basal rate profile (4, 6, 7) over the input unit of the insulin pump, wherein each node point (5, 5') is defined by a start time of a basal rate delivery and a basal rate to be delivered associated with this start time or an amount of insulin to be delivered associated with this start time,
- Formation of a continuous function related to the node points (5, 5') by means of interpolation and/or approximation by the calculation unit of the insulin pump, wherein the continuous function maps and connects the node points (5, 5') to one another, and
- Production from the continuous function of a time sequence (4, 6, 7) of basal rates to be delivered by the insulin pump during specified time sections by means of the calculation unit of the insulin pump by discretizing the continuous function, wherein, during the discretization process, the time period of the continuous function is divided into a finite number of adjoining time sections and a value of the continuous function is assigned to each time section.

2. The method as claimed in claim 1, **characterized in that**, in addition to the node points (5, 5'), the continuous function also maps stored basal rates.

3. The method as claimed in claim at 1 or 2, **characterized in that** four to ten node points (5, 5') are defined.

4. The method as claimed in one of the preceding claims, **characterized in that** at least 24 basal rates are produced.

5. The method as claimed in one of claims 1 to 3, **characterized in that** less than 24 basal rates are produced, wherein the number of basal rates produced is greater than the number of node points (5, 5') defined.

6. The method as claimed in one of the preceding claims, **characterized in that**, when forming the continuous function, the last node point in time and the first node point in time are directly connected to one another by the continuous function so that discontinuous basal rate delivery does not occur.

7. The method as claimed in one of the preceding claims, **characterized in that** the number of sign changes of the derivation of the continuous function is determined by the calculation unit and, in the event that the number of sign changes exceeds a predefined limit, a warning signal is produced.

8. The method as claimed in one of the preceding claims, **characterized in that** the defined node points (5, 5') are compared with predefined node points and/or stored basal rates of a stored basal rate profile and, only in the event that the defined node points (5, 5') deviate by no more than a predefined value, in particular by no more than 20%, from the predefined node points and/or stored basal rates, a continuous function related to the defined node points (5, 5') and, if applicable, stored basal rates, which maps the defined node points (5, 5') and, if applicable, stored basal rates, is formed by means of the calculation unit of the insulin pump, and a time sequence (4, 6, 7) of basal rates to be delivered by the insulin pump during specified time sections is produced from the continuous function by means of the calculation unit of the insulin pump.

9. The method as claimed in one of the preceding claims, **characterized in that** the defined node points (5, 5') are compared with predefined node points and/or stored basal rates and, in the event that the defined node points (5, 5') deviate by more than a predefined value, in particular by more than 20%, from the predefined node points and/or stored basal rates, a warning signal is produced.

## Revendications

1. Procédé pour établir un profil des doses de base (4, 6, 7) pour une pompe à insuline comprenant une unité d'entrée et une unité de calcul, **caractérisé par** les étapes suivantes :
- définition d'un nombre de positions d'appui (5, 5') pour le profil des doses de base (4, 6, 7) au moyen de l'unité d'entrée de la pompe à insuline, chaque position d'appui (5, 5') étant définie par un temps de départ d'une délivrance de dose de base et par une dose de base à délivrer correspondant à ce temps de départ, ou une quantité d'insuline à délivrer correspondant à ce temps de départ ;
- formation d'une fonction continue correspondant aux positions d'appui (5, 5), par interpolation et/ou approximation, par l'unité de calcul de la pompe à insuline, la fonction continue représentant les positions d'appui (5, 5') et les reliant entre elles, et
- production d'une série ordonnée (4, 6, 7) de doses de base qui doivent être délivrées par la pompe à insuline pendant des périodes déterminées, sur la base de la fonction contiune par l'unité de calcul de la pompe à insuline, la fonction continue étant discrétisée, la plage de temps de la fonction continue étant divisée en un nombre fini de périodes consécutives lors de la discrétisation et une valeur de la fonction continue étant attribuée à chaque période.

2. Procédé selon la revendication 1, **caractérisé par le fait qu'**en supplément des positions d'appui (5, 5'), la fonction continue représente des doses de base mémorisées.

3. Procédé selon l'une des revendications 1 ou 2, **caractérisé par le fait que** quatre à dix positions d'appui (5,5') sont définies.

4. Procédé selon l'une des revendications précédentes, **caractérisé par le fait qu'**au moins 24 doses de base sont produites.

5. Procédé selon l'une des revendications 1 à 3, **caractérisé par le fait que** moins de 24 doses de base sont produites, le nombre des doses de base produites étant plus grand que le nombre des positions d'appui (5, 5') définies.

6. Procédé selon l'une des revendications précédentes, **caractérisé par le fait que**, lors de la formation de la fonction continue, la position d'appui qui est la dernière par son temps et la position d'appui qui est la première par son temps sont liées directement entre elles par la fonction continue, de telle sorte qu'il ne se produit pas de délivrance instable de doses de base.

7. Procédé selon l'une des revendications précédentes, **caractérisé par le fait que** le nombre de changements de signe de la dérivée de la fonction continue est déterminé par l'unité de calcul et, dans le cas où le nombre de changements de signe excède une valeur limite prédéfinie, un signal d'avertissement est produit.

8. Procédé selon l'une des revendications précédentes, **caractérisé par le fait que** les positions d'appui définies (5, 5') sont comparées à des positions d'appui prédéfinies et/ou à des doses de base mémorisées appartenant à un profil de doses de base mémorisé, et **par le fait que** ce n'est que dans le cas où les positions d'appui définies (5, 5') ne diffèrent pas de plus d'une valeur prédéfinie, en particulier ne diffèrent pas de plus de 20 pour cent, des positions d'appui prédéfinies et/ou des doses de base mémorisées, qu'une fonction continue correspondant aux positions d'appui définies (5, 5') et aux doses de base éventuellement mémorisées, qui reproduit les positions d'appui définies (5, 5') et les doses de base éventuellement mémorisées, est formée au moyen de l'unité de calcul de la pompe à insuline, et qu'une série ordonnée (4, 6, 7) de doses de base à délivrer par la pompe à insuline pendant des périodes déterminées est produite au moyen de l'unité de calcul de la pompe à insuline sur la base de la fonction continue.

9. Procédé selon l'une des revendications précédentes, **caractérisé par le fait que** les positions d'appui définies (5, 5') sont comparées à des positions d'appui prédéfinies et/ou à des doses de base mémorisées et **par le fait que**, dans le cas où les positions d'appui définies (5, 5') diffèrent de plus d'une valeur prédéfinie, en particulier de plus de 20 %, des positions d'appui prédéfinies et/ou des doses de base mémorisées, un signal d'avertissement est produit.
